# EUROPEAN PATENT APPLICATION

(11) **EP 1 231 262 A1**
(43) Date of publication of application: **14.08.2002**
(21) Application number: 00969994.3
(22) Date of filing: 23.10.2000
(51) Int. Cl.: C12N 5/08, C12N 15/12, A61K 35/14, A61P 35/00

(54) **METHOD OF IN VITRO CULTURE OF LYMPHOCYTES AND GENE THERAPY COMPOSITIONS**

(30) Priority: 21.10.1999 JP 30012299
(71) Applicant: Teshigawara, keisuke, Kyoto-shi, Kyoto 606-0911 (JP); Ohkubo, Yuji, Kyoto-shi, Kyoto 604-0000 (JP)
(72) Inventor: Teshigawara, keisuke, Kyoto-shi, Kyoto 606-0911 (JP); Ohkubo, Yuji, Kyoto-shi, Kyoto 604-0000 (JP)
(74) Representative: Clisci, Serge
(86) International application number: JP0007385
(87) International publication number: WO0129191

(57) **Abstract**

The method for *in vitro* culture of lymphocytes involves incubating lymphocytes and cells with a particular gene expressed in a particular cancer cell line or cells, which are deficient or lowered in expression of a class 1 antigen and the particular gene has already been expressed, to amplify mainly NK cells or non-MHC-bound or MHC-bound killer T cells and then to amplify killer T cells specific to an antigen to a cancer. The *in vitro* culture of the lymphocytes can produce a group of lymphocytes composed mainly of killer cells.

The group of the lymphocytes so produced can be used for immune therapy effective even for patients with cancer in the terminal stage to whom conventional cancer treatment and cancer curing agents.

## Description

### TECHNICAL FIELD

The present invention relates to a novel method of *in vitro* culture for the multiplication of lymphocytes and a novel composition for use in immune therapy by using amplified lymphocytes. More particularly, the present invention relates particularly to a composition for use in immune therapy, which can realize a remarkably useful cancer therapy that can be effective even for patients with cancer ineffective by conventional cancer therapy.

### TECHNICAL BACKGROUND

In order to culture solely a lymphocyte group consisting of NK cells for a long period of time under *in vitro* circumstances, there have hitherto been used mainly two methods. (1) One method involves using autolougous B cells for the maintenance of culturing NK cells, the B cells being modified by EB virus and then irradiated with radiation to repress the multiplication of NK cells. This known method can maintain the culture of NK cells, but it requires purification of NK cells and establishment of autolougous B cell line. (2) The other method involves culturing NK cells from lymphocytes with IL2 without purification. The resulting NK cells have been used for LAK therapy. This method, however, can multiply the NK cells by several times only and the effects achieved by this therapy are limited.

Further, the method (1) suffers from the problems that it has to use EB virus known as virus involved with oncogenesis and that the therapy using a lymphocyte group consisting of such NK cells may cause serious side effects, etc. Moreover, the modified B cells vary to a great extent in ability concerning therapy effects so that it is very difficult to achieve stable culture. In addition, this method requires purification of NK cells so that a loss of cells is caused and a great amount of labor is required.

On the other hand, the method (2) has the defects that it can multiply NK cells by several times only so that the ability of multiplying NK cells is low and, if the culture would have been carried out for a long period of time, T cells having no killer activity may be caused to multiply selectively, therefore, the therapy effects have to become limited.

With the above background taken into account, the present inventors have conducted extensive review and studies on a method for *in vitro* culturing lymphocytes which can stably produce a lymphocyte group effective for cancer therapy yet which does not use virus such as EB virus, etc. involved with oncogenesis, and which does not require purification of NK cells. As a result, the present inventors have found a novel method for *in vitro* culturing lymphocytes by using an approach that is thoroughly different from conventional methods for culturing NK cells and that can produce such a lymphocyte group stably. More specifically, it has been found that this method can multiply a lymphocyte group consisting of activated NK cells and CD4-positive T cells in a safe and stable manner and that such a lymphocyte group can be used as a source of conventional adoptive immune therapy. It is further found that the NK cells grown by this method have the activity of damaging cancer cells higher than those grown by conventional methods.

On the basis of the studies performed, the present inventors have previously proposed an *in vitro* culture method for multiplying a lymphocyte group having a high killer activity concerning a cancer cell-damaging activity by culturing a combination of class I-negative ( or the expression of class I is low ) cancer cells with B7 gene expressed therein and lymphocytes derived from peripheral blood with an immunomodulator at various rates (Japanese Patent Application No. 59,336/1999; Laid-open No. ). It now has been found that this *in vitro* culture method still has the points to be improved that a lymphocyte group having a high killer activity can be multiplied by several times, but that killer cells selectively derived from such a lymphocyte group so as to be adapted to individual patients cannot be multiplied to more than 10 times.

### DISCLOSURE OF THE INVENTION

With the above situation taken into account, the present inventors have continued extensive review and studies to find a method for *in vitro* culturing lymphocytes which can selectively derive killer cells having a killer activity so as to adapt to each individual and multiply such killer cells. As a result, the present inventors have found a method for *in vitro* culturing lymphocytes which can multiply NK cells or non-MHC-bound or MHC-bound killer cells in combination with killer T cells specific to cancer antigen. It was found that this *in vitro* culture method can selectively derive and multiply killer cells from the lymphocytes having a killer activity so as to adapt to individual patients without purifying specific precursor cells. It was further found that the killer cells cultured and multiplied by this *in vitro* culture method can be applied as a source of an immune therapy even for patients with cancer for whom conventional cancer therapy was ineffective, so that this method can realize a remarkably effective cancer therapy. The present invention has been completed based on these findings.

Therefore, the present invention has the object to provide a method for the *in vitro* culture of lymphocytes that can selectively derive killer cells from lymphocytes having a killer activity and multiply such killer cells. The present invention has another object to provide a composition for use in immune therapy which comprises the killer cells cultured and multiplied by the *in vitro* culture method and which can be applied as a source of immune therapy.

In order to achieve the above objects, the present invention provides a method for the *in vitro* culture of lymphocytes, which comprises culturing lymphocytes and a cell with B7 gene expressed in a particular cancer cell or with B7 gene and a particular gene such as a cancer-antigen gene expressed therein or a cell with such a gene already expressed therein to multiply mainly NK cells or non-MHC-bound or MHC-bound killer T-cells; and multiplying killer T cells specific to a cancer antigen together with the NK cells or the non-MHC-bound or MHC-bound killer T cells.

In a preferred embodiment of the present invention, there is provided an *in vitro* culture method for growing lymphocytes which involves using cancer cells, as the particular cancer cells, which are deficient or low in the expression of a class 1 antigen. Further, in a preferred embodiment, the present invention provides a method for *in vitro* culture of lymphocytes, which involves using the expression gene consisting of B7 gene or a mixture of B7 gene with a cancer antigen gene or gene(s) of cell adhesion molecules In a more preferred embodiment, the present invention provides a method for the *in vitro* culture of lymphocytes, which involves using lymphocytes immediately after the separation from peripheral blood or lymphocytes activated with an immunomodulator that can facilitate damaging cancer cells.

In addition to the culture method for the *in vitro* growth of the lymphocytes, the present invention in another aspect provides a composition for use in immune therapy, which comprises lymphocytes obtained by amplifying NK cells or killer T cells produced by the *in vitro* culture method according to the one aspect of the present invention.

Other objects, features and advantages will become apparent in the course of the following description.

### BRIEF DESCRIPTION OF THE ACCOMPANYING DRAWING

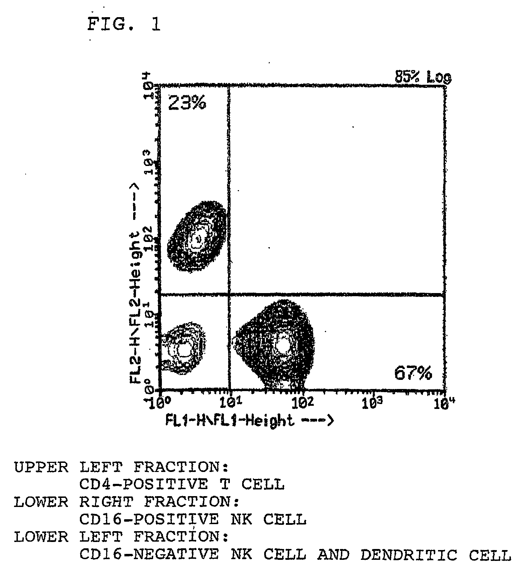

FIGURE 1 shows the relationship of NK cells with killer T cells specific to a cancer antigen.

### BEST MODES FOR CARRYING OUT THE INVENTION

The method for *in vitro* culturing lymphocytes according to the present invention involves amplifying mainly non-MHC-bound or MHC-bound killer T cells by culturing lymphocytes in admixture with a cancer cell in which a particular gene is caused to be expressed therein or a cell in which such a particular gene has been expressed therein, such a particular gene including, for example, B7 gene or a combination of B7 gene with a cancer antigen gene, and further amplifying killer T cells specific to a cancer antigen.

The lymphocytes to be used as an effecter for the present invention may be collected from patients with cancer (malignant lymphoma, liver cancer, pancreas liver, large intestine cancer, etc.) through leukophresis or intravenous puncture. The blood collected directly from patients is usually processed by conventional methods to separate lymphocytes and adjusted with RPMI or the like to a predetermined concentration, e.g., 2 x 10⁶ cells per milliliter. The measurement of the concentration of cells may be conducted with a hemocytometer. A suspension of the lymphocytes with their concentration adjusted to such a predetermined level is used as a lymphocyte stock suspension for use in the *in vitro* lymphocyte culture. The steps for procedures are similar to conventional ones. The procedures for the method of the present invention may be carried out by preparing a culture medium by diluting the lymphocyte stock suspension to a predetermined optimal concentration.

In accordance with the present invention, as the cells to be used for the *in vitro* culture of lymphocytes for the expression of the particular gene, there may be used a cancer cell line that is deficient or decreased in the expression of a class 1 antigen. Such a cancer cell line may include, for example, K 562 cell derived from human chronic myelocytic leukemia and Daudi cell derived from lymphoma.

Further, the cancer antigen gene may be collected from cancer cells of a patient with cancer by means of conventional methods such as a stab suction method and identified by means of conventional methods such as PCR method. For instance, the cancer antigen gene may be identified by extracting RNA from the collected cancer cells by means of acid-guanidium thiocyanate/phenol-chloroform extraction method (AGPC method) and detecting the expression of the gene by means of PCR method or DNA chips, etc. using cDNA obtained by RT (Reverse Transcription) reaction as a template.

As the method for the introduction of the particular gene into the cancer cells, there may be used conventional introduction methods which may include, for example, microinjection method, phosphate calcium-DNA co-precipitation method, DEAE-dextran method, liposome method, particle gun method, electroporation method, gene introduction method using a microorganism such as retro virus, adenovirus, herpes virus, protoplast fusion or cell fusion method, and so on. For the method for the expression of the gene introduced into the target cancer cell line, such conventional expression methods can be used.. For instance, an expression gene may be bound to an expression vector with an gene resistant to an toxic agent such as neomycin or hygromycin and introduced into the target cells by means of conventional methods such as electroporation method or the like. After the gene has been introduced, the agent such as neomycin, hygromycin or the like is added to kill the cells other than the cells with the target gene introduced therein and to collect only the cells with the target gene expressed therein. The collected cells with the target gene expressed therein are then incubated and amplified by using conventional methods such as limiting dilution-culture method or the like and the amplified cells are used for *in vitro* culturing lymphocytes. A plurality of genes can also be expressed simultaneously by means of the like method. In order to use plural expression genes, accordingly, it is preferred to use cells in which the plural expression genes are expressed simultaneously. Such gene-expression cells are used as feeder cells.

The method of the *in vitro* culture of the lymphocytes in accordance with the present invention may be carried out by diluting a lymphocyte culture medium prepared to an optimal concentration with a predetermined concentration of the lymphocyte culture stock suspension, adding the mitomycin-treated or irradiated feeder cells at a predetermined rate to the culture medium and incubating the lymphocytes in the culture medium for a predetermined period of time. This culture does not require special operations and can be done in accordance with conventional procedures. More specifically, the feeder cells are diluted to 2 x 10⁴ cells per milliliter with a lymphocyte culture adjustment liquid prepared by diluting the lymphocyte culture stock suspension and cultured for a week or longer in a CO₂ incubator. This culture allows the amplification of mainly NK cells or non-MHC-bound or MHC-bound killer T cells as well as killer T cells specific to the cancer antigen. This allows the amplification of killer cells having the activity to damage the cancer cells to approximately several ten times to thousand times or more by co-culturing the cancer cell line to be added at an appropriate frequency. After culturing, the culture liquid is washed with PBS to purify amplified killer cells. The killer cells so cultured and amplified are then returned to the patient, from which the lymphocytes were gathered, by administering them through a dripping pack or the like.

As the lymphocytes, there may be used lymphocytes separated from peripheral blood just after drawing and lymphocytes activated with an immunomodulator that can become likely to damage the cancer cells. As the immunomodulators, there may be mentioned, for example, various cytokines, various biologically response modifiers, various herbaceous materials, nutritious materials contained in various food, or trace metals that may give an influence on an intercellular machinery. These immunomodulators may be used singly or in combination of two or more. Such various cytokines to be used for the present invention may include, for example, IL-1 to IL-18, inclusive, TNF-., TNF-. , INF-.. INF-.. INF-. G-CSF, M-CSF, GM-CSF and so on. Further, such various biological response modifiers may include, for example, OK 432 (Picibanil™, Chugai Pharmaceuticals, Co., Ltd., Japan), sizofiran (Soniflan™, Kaken Pharmaceutical, Co., Ltd.), urinastatin (Miraclid™, Mochida Pharmaceuticals, Co., Ltd.), lentinan, Maruyama vaccine and so on. Moreover, the immunomodulators including such various herbaceous materials, nutritious materials and trace metals are illustrated specifically in Japanese Patent Application Laid-open No. 11-300,122 which is incorporated by reference as part of this description. The immunomodulators may generally be used in the form of an extract liquid or, as needed, in the form of concentrated extract liquid, obtained by drying or pulverizing them by conventional methods that do not damage their components, and extracting them with water or other solvents. Such extract liquid is then admixed with a culture liquid for culturing the lymphocytes.

A detailed description will be given regarding the *in vitro* culture method for lymphocytes according to the present invention by way of working examples. It is to be noted herein, however, that the present invention is not limited in any respect to those working examples. Further, in the following specification, the B7 gene is described as an expression gene, but it should be understood that the present invention is not intended at all to be limited to the B7 gene.

### Examples:

### Procedures of incubating cancer cells with lymphocytes with the B7 gene expressed therein;

The B7 gene was incorporated into an expression vector having a neomycin-resistant gene, and the expression vector was introduced into K562 cells by means of electroporation method in order to allow the B7 gene to be expressed therein. The K562 cells with the B7 gene expressed therein and the human lymphocytes were cultured in a medium (Hi-Medium™: NIPRO) containing IL-2 to which human serum was added, and they were incubated under 5% CO₂ at 37°C. Although the amount of the culture medium may vary with the amount of the lymphocytes, the human lymphocytes were adjusted to 1 - 5 x 10⁶ cells per milliliter in this example. And the K562 cells with the B7 gene expressed therein were added at the rate of 1/100 to 1/500. Further, the B7-expression cells were treated with mitomycin or by irradiation so as to cause no amplification. The B7-expression cells were cloned by the limiting dilution-culture method and the cells having high expression were selected by means of flow rate meter.

### Procedures of separating lymphocytes as an effector:

The blood drawn from a patient was poured in a centrifugal tube and diluted to twice with a phosphate buffered solution (PBS), followed by transferring the blood into plural lympho-prep tubes for use in blood separation and centrifuged at 1,500-2,000 rpm at ambient temperature for 15 to 20 minutes. The resulting lymphocyte layer (a layer so-called as buffy coat) was gathered and transferred into a new centrifugal tube and washed with about 30-40 ml per tube of PBS or RPMI 1640, followed by centrifugation at 1,500 rpm for 10 minutes and removal of the resulting supernatant. This operations were repeated twice and a human serum-added medium (Mi-Medium™) was added thereto to adjust the lymphocytes to a predetermined concentration, e.g., 4 x 10⁶ cells per milliliter. The measurement of the lymphocyte concentration was conducted with a blood count plate and the number of lymphocytes was counted as accurately as possible. The resulting liquid was used as a lymphocyte culture stock liquid.

The lymphocyte culture stock liquid was incubated in substantially the same manner as the above incubation. For example, the culture was carried out by pouring 5 ml of the lymphocyte culture stock liquid into each flask to adjust the number of the feeder cells to 4 x 10⁴ cells per milliliter, incubating the flask in a CO₂ incubator for one week according to conventional method, washing the lymphocyte culture medium with physiologically saline, and selecting the lymphocytes followed by purification.

The lymphocytes purified in the above manner are then subjected to investigation according to the methods as proposed in our copending Japanese Patent Application Nos. 9-342,675 and 11-174,053. The results of investigation are shown in Table 1 below and reveal that the ability of the lymphocytes for the damaging of the cancer cells was increased by approximately 9 times.

Now, a brief description will be given regarding the method of investigation. A lymphocyte culture medium was prepared by separating the lymphocytes separated from peripheral blood and adding the lymphocytes to a culture medium, followed by incubating the culture medium overnight in an incubator. The lymphocytes so incubated are used as an effecter and the magnitude of Europium radiated from the cells is measured by using K562 cells labeled with Europium as a target by means of fluorophotometer. The results are shown in Table 1 below.

**TABLE 1:**

| Comparison of the immune ability of lymphocytes at the time of gathering blood (A) with the immune ability of lymphocytes after the application of this invention (B) | | | | |
|---|---|---|---|---|
| IMMUNE ABILITY (%) | RATIO | | | |
| | 40:1 | 20:1 | 10:1 | 5:1 |
| (A) | 5.5 | 3.2 | 1.5 | 1.3 |
| (B) | 96.3 | 80.5 | 70.5 | 55.8 |

As is apparent from Table 1 above, the lymphocytes prepared by the culture method according to the present invention are found to be a group of lymphocytes, as is shown in Fig. 1, which consist mainly of killer cells having a high activity of damaging the cancer cells. The results as shown in Fig. 1 are those obtained by the measurement with a FACScan flowcytometry (Beckton-Dickinson). From these results, it is indicated that the group of the resulting lymphocytes is composed mainly of NK cells and killer T cells specific to cancer cells.

### EFFECTS OF THE INVENTION

As is described above, the method of the *in vitro* culture of lymphocytes can amplify a group of lymphocytes composed mainly of NK cells and/or killer T cells specific to cancer in a high yield and in a stable manner by incubating a mixture of lymphocytes with cells at a predetermined rate, in which a particular expression gene such as B7 gene or a cancer antigen gene has been introduced in particular cancer cells and the particular expression gene has been expressed or in which such a particular expression gene has already been expressed. The lymphocytes group presents the extremely great merits that it can be used as a source of an effective immune treatment even for cancer patients to which conventional cancer therapy has been found ineffective, so that the present invention can realize a remarkably effective cancer treatment.

The group of the lymphocytes prepared by the *in vitro* culture according to the present invention can provide the great merits that it can be used for the immune treatment for cancer as an reagent for immunologically treating cancer, which does not involve oncogenesis, in place of the conventional method that uses B cells mutated by virus involved with oncogenesis.

Further, the method for the *in vitro* culture of the lymphocytes according to the present invention can selectively induce and amplify killer cells from the lymphocytes having killer activity, which adapt to the individual patient. Therefore, particularly through effective destruction of cancer cells, the killer T cells specific to the cancer antigen can be also amplified in a stable way so that the present invention can be applied as a source of an effective immune therapy even for the patients with cancer, to which the conventional cancer therapy has been ineffective, so that the present invention can provide the great merits that it can realize a remarkably effective cancer treatment.

## Claims

1. A method for the *in vitro* culture of lymphocytes comprising incubating lymphocytes and cells in which a particular gene has been expressed in a particular cancer cell or in which such a particular cancer has already been expressed, to amplify mainly NK cells or non-MHC-bound or MHC-bound killer T cells and further to amplify killer T cells specific to a cancer antigen.

2. The method for the *in vitro* culture of the lymphocytes as claimed in claim 1, wherein said cancer cells comprise cancer cells which are deficient or lowered in expression of a class 1 antigen.

3. The method for the *in vitro* culture of the lymphocytes as claimed in claim 1 or 2, wherein said particular gene is B7 gene or a combination of B7 gene with cancer antigen gene or a gene for a cell-binding factor.

4. The method for the *in vitro* culture of the lymphocytes as claimed in any one of claims 1 to 3, wherein said lymphocytes are those collected immediately after separation from peripheral blood or those activated by an immunomodulator that can become likely to damage a cancer cell.

5. A composition for use in immune therapy, comprising lymphocytes obtained by amplifying mainly NK cells or non-MHC-bound or MHC-bound killer T cells by incubating lymphocytes and cells with a particular gene expressed in a particular cancer cell or cells in which said particular gene has already been expressed, and amplifying killer T cells specific to a cancer antigen by means of the method for the *in vitro* culture of the lymphocytes as claimed in any one of claims 1 to 4.

6. The composition for use in immune therapy as claimed in claim 5, wherein said cancer cells comprise cancer cells which are deficient or lower in expression of a class 1 antigen.

7. The composition for use in immune therapy as claimed in claim 5 or 6, wherein said particular gene is B7 gene or a combination of B7 gene with cancer antigen gene or a gene for a cell-binding factor.

8. The composition for use in immune therapy as claimed in any one of claims 5 to 7, wherein said lymphocytes are those collected immediately after separation from peripheral blood or those activated by an immunomodulator that can become likely to damage a cancer cell.
